# EUROPÄISCHE PATENTSCHRIFT

(11) **EP 0 700 275 B1**
(45) Veröffentlichungstag und Bekanntmachung des Hinweises auf die Patenterteilung: **16.07.1997**
(21) Anmeldenummer: 94916873.6
(22) Anmeldetag: 27.05.1994
(51) Int. Cl.: A61F 6/14, A61F 6/18

(54) **KOMBINATION AUS EINER INTRA-UTERIN EMPFÄNGNISVERHÜTENDEN VORRICHTUNG UND AUS EINEM EINFÜHRUNGSGERÄT**
COMBINATION OF AN INTRA-UTERINE CONTRACEPTIVE DEVICE AND AN INSERTION DEVICE
DISPOSITIF ANTICONCEPTIONNEL INTRA-UTERIN ET DISPOSITIF D'INSERTION COMBINES

(30) Priorität: 27.05.1993 DE 4318941
(43) Veröffentlichungstag der Anmeldung: 13.03.1996
(73) Patentinhaber: SCHERING AKTIENGESELLSCHAFT, 13342 Berlin (DE)
(72) Erfinder: AUGUSTIN, Rainer, D-79258 Hartheim-Bremgarten (DE)
(86) Internationale Anmeldenummer: DE9400620
(87) Internationale Veröffentlichungsnummer: WO9427531

(56) Entgegenhaltungen:
- DE-A- 2 328 409
- DE-A- 3 222 795
- FR-A- 2 261 744
- FR-A- 2 385 410
- GB-A- 782 496
- US-A- 3 783 861

## Beschreibung

Die Erfindung betrifft eine Kombination einer intra-uterin empfängnisverhütenden Vorrichtung und eines entsprechenden Einführungsgerätes, das zum Einbringen der Vorrichtung in den Uterus verwendet wird.

### Stand der Technik

In der EP 0 011 143 wird eine Kombinationen aus einer intra-uterin empfängnisverhütenden Vorrichtung und einem der Vorrichtung entsprechenden Einführungsgerät beschrieben. Die Vorrichtung besitzt einen Körper, zwei elastische Arme, die an dem cranialen Ende des Körpers angeordnet sind, und einen Befestigungsteil am caudalen Ende des Körpers. Die beiden elastischen Arme stehen im wesentlichen seitlich von dem Körper ab, lediglich im zusammengelegten Zustand liegen beide Arme parallel. Im zusammengelegten Zustand befinden sich die Arme unter elastischer Spannung. Der Befestigungsteil weist ein Auge auf, an dem zwei Kunststoffäden befestigt sind. Diese Kunststoffäden dienen dazu, das Pessar beim Entfernen aus dem Uterus herausziehen zu können. Das Einführungsgerät setzt sich aus einem Hohlstab und einem Stempel mit einer Stempelhalterung zusammen. Der Hohlstab trägt auf seiner Außenseite eine Markierung. Der Hohlstab wird von einen hohlzylinderförmigen Schieber umgeben, der gleitend der Außenwand des Hohlstabs anliegt. Der Schieber dient dazu, die von dem Arzt zuvor mit Hilfe einer Sonde ermittelte Distanz zwischen Fundus uteri und dem Muttermund einstellen zu können. Der Schieber zeigt somit beim Einlegevorgang dem Arzt an, wann er Gefahr läuft, mit Hilfe der Kombination an den Fundus uteri zu stoßen. Die Stempelhalterung, die am caudalen Ende des Stempels angeordnet ist, besitzt eine kreisförmige Gestalt. Im caudalen Bereich besitzt der Hohlstab und der Stempel einen gemeinsamen Haftbereich, in dem beide (Hohlstab und Stempel) schwergängig gegeneinander verschiebbar sind.

Aus der Druckschrift DE 34 11 768 ist ein eine Kombination bekannt, mit der intra-uterin empfängnisverhütende Metallspiralen in Form einer Wendel in die Wandung des Uterus eindrehbar sind. Eine solche Kombination besitzt eine Federung, die nach Berührung der Uteruswand die Wendel in eine für die Eindrehung vorteilhafte Lage zu dem Einführgerät bewegt.

Verwendet der behandelnde Arzt die Kombination nicht Vorschriftsmäßig, können Verletzungen, insbesondere des Fundus uteri, während des Einlegevorganges auftreten. Nachteilig ist weiterhin, daß der Arzt Gefahr laufen kann, die Vorrichtung nicht in der wünschenswerten Position einzulegen. Die abgespreizten Arme des Pessars sollen in das Lumen der Tuben des Uterus ragen. Haben sich die Arme verdreht und weichen sie von dieser wünschenswerten Position ab, kann es zu Verletzungen der Uteruswand oder zu einem falschen und schmerzhaften Sitz des Pessars kommen. Beim Einführen der Vorrichtung besitzt der Arzt keine sichere Kontrolle darüber, das Pessar in der gewünschten Richtung ausrichten zu können.

### Aufgabe

Es ergibt sich somit die Aufgabe, eine Kombination gemäß dem Stand der Technik dahingehend abzuändern, daß die Handhabung der Kombination durch den Arzt erleichtert wird, die Einlegetechnik sicherer gemacht wird und eine korrekte Lage der intra-uterin empfängnisverhütenden Vorrichtung ermöglicht wird. Insbesondere wird darauf abgestellt, die Verletzungsgefahr bei dem Einlegevorgang zu minimieren.

### Lösung der Aufgabe

Die Aufgabe wird durch eine Kombination gelöst, die aus einer intra-uterin empfängnisverhütenden Vorrichtung und einem dieser Vorrichtung entsprechenden Einführungsgerät mit folgenden Merkmalen besteht:
a) die intra-uterin empfängnisverhütende Vorrichtung besitzt einen Körper, an dessen cranialem Ende wenigstens zwei elastische Arme angeordnet sind und dessen caudales Ende ein Befestigungsteil bildet, an das eine Vorrichtung zur Entfernung der intra-uterin empfängnisverhütenden Vorrichtung aus dem Uterus befestigbar ist;
b) das Einführungsgerät besteht aus einem Hohlstab und einem im Hohlstab bewegbaren Stempel, der am caudalen Ende wenigstens eine Stempelhalterung aufweist;
c) im caudalen Bereich ist der Hohlstab und/oder der Stempel als Federung ausgebildet;
d) am caudalen Ende des Hohlstabs und im caudalen Bereich des Stempels cranial von der Stempelhalterung sind Hohlstab und Stempel in einem Haftbereich gleitend oder fest miteinander verbunden;
e) zwischen Stempel und intra-uterin empfängnisverhütender Vorrichtung und/oder cranial von der vollständig in den Hohlstab eingezogenen intra-uterin empfängnisverhütenden Vorrichtung befindet sich ein in radialer Richtung von dem Hohlstab umgebener Hohlraum als Federzone innerhalb der bei einer Relativbewegung von Stempel und Hohlstab zueinander die Federung des Hohlstabs anspricht und die intra-uterin empfängnisverhütende Vorrichtung noch vollständig im Einführungsgerät bleibt.

### Vorteile

Dabei kann das craniale Ende des Hohlstabs über die Enden der zusammengefalteten Arme der Vorrichtung, die vollständig in dem Hohlstab eingezogen ist, hinausragt. Dadurch ergibt sich eine Federzone cranial von der Vorrichtung. Bevorzugt ist eine Federzone, die zwischen Stempel und Vorrichtung liegt. Die Federzone wird aufgehoben, indem die Federung aktiviert wird.

Eine derartige Kombination hat gegenüber dem Stand der Technik verschiedene Vorteile.

Wenn das craniale Ende des Hohlstabs abgerundete Ecken aufweist, ist es nicht gefährlich, wenn dieses craniale Ende des Hohlstabs den Fundus uteri berührt. Hierdurch entsteht bei sanftem Druck nicht die Möglichkeit, das Gewebe des Fundus uteri aufzureißen. Der Druck, der hierbei von dem cranialen Ende des Hohlstabs auf den Fundus uteri ausgeübt wird, wird durch die Rückstellkraft der Federung kontrolliert. Erstrebenswert ist es, die Federung schon auf leichten Druck hin ansprechen zu lassen. Für den Hersteller ergeben sich zwei Grenzbereiche: Die Rückstellkraft darf nicht so weich sein, daß ein Durchdringen des Muttermundes und ein Vorschieben des Hohlstabs allein schon zum Ansprechen der Feder führt und weiterhin darf die Federkraft nicht so stark ausgeprägt sein, daß eine Ruptur des Fundus uteri möglich ist. Das herausfinden der für die Praxis notwendigen Federkraft ist für den Fachmann eine leicht zu bewerkstelligende Aufgabe. Beim Einführen der Kombination ist es für den Arzt von Bedeutung, daß er die Schubkraft für das Einführen der Kombination caudal von der Federung einwirken läßt.

Dieses gegenüber dem Stand der Technik für die Frau gefahrlosere Einführungsverfahren erlaubt es dem Arzt, das Gerät ruhiger und besonnener einführen zu können. Dieses hat zur Folge, daß eine ungewollte Drehung der Kombination um die Längsachse vermieden wird, so daß auch eine korrekte Positionierung der Arme in Richtung der Tuben des Uterus die Folge ist. Ein weiterer, wesentlicher Vorteil besteht darin, daß die Entfaltung des Pessars ebenfalls eine Verletzungsgefahr ausschließt. Hierfür sorgt die räumliche Gestalt der erfindungsgemäßen Kombination. Der Hohlstab kann von dem Arzt nicht weiter in Richtung des Fundus uteri gestoßen werden, wenn er den Hohlstab caudal von der Federung anfaßt. Das entfalten des Pessars ist mit einem Zurückziehen des Einführungsgerätes aus dem Uterus verbunden.

Die Stempelhalterung ist bevorzugt halbkreisförmig ausgebildet. Sie soll es dem Arzt erlauben, mit Hilfe des Daumens einen Druck auf den Stempel ausüben zu können. Dabei ist besonders hervorzuheben, daß der Daumen sich nicht aktiv in Richtung des Fundus uteri bewegt, sondern lediglich die Gegenkraft halten muß, die durch das Zurückziehen des Hohlstabs entsteht. Der Arzt soll und kann bei diesem Einlegevorgang lediglich eine Zugbewegung und Zugkraft aufwenden. Die Federung hat die Aufgabe, eine Verletzung nahezu unmöglich zu machen. Die Federzone muß bezüglich der Rückstellkraft so ausgelegt sein, daß sie durch die Gleitreibung noch nicht gestaucht wird, die bei dem Durchdringen und der Passage des Muttermundes auftritt. Jedoch sollte eine Stauchung dann ablaufen, wenn der Fundus uteri von dem cranialen Ende des Hohlstabs berührt wird. Die Federung, die auf Grund dieser Berührung eine Stauchung erfährt, zeigt dadurch gleichzeitig dem Arzt an, daß er mit dem Hohlstab den Fundus uteri berührt hat. Je nach Ausführungsform des Einführungsgerätes ist es sinnvoll, daß der Teil des Hohlstabs, der caudal von der Federung liegt, gegenüber dem Stempel gleitend ist oder mit dem Stempel kraftschlüssig verbunden ist. Führt der Arzt die Kombination ein, indem er die Kombination in dem Bereich des Hohlstabs anfaßt, der caudal von der Federung liegt, kann der Haftbereich gleitend sein. Die Gleitreibung muß lediglich so stark ausgeprägt sein, daß ein Verschieben des Stempels auf Grund der Schwerkraft nicht erfolgt. Diese Wirkung kann auch durch eine Federung erzeugt werden, die ein Verschieben des Stempels auf Grund der Schwerkraft gegenüber dem Hohlstab nicht erlaubt. Es ist jedoch auch möglich, daß der caudale Bereich des Hohlstabs die Stempelhalterung oder eine andere Verdickung des Stempels berührt. Diese Verdickung kann z.B. eine Schulter sein, auf der dieser caudale Bereich aufliegt. In einem solchen Fall schiebt der Arzt vor dem Einlegen der Kombination die Vorrichtung, den Stempel und den Hohlstab so zusammen, daß der caudale Bereich des Hohlstabs direkt auf der Schulter oder der Stempelhalterung aufliegt. Der Arzt führt nun die zusammengelegte Kombination in den Uterus ein, indem er sowohl den caudalen Bereich des Hohlstabs jenseits der Federung als auch Stempel samt Stempelhalterung umfaßt.

Um die Federung des Hohlstabs nutzen zu können, ist es erforderlich, daß bei einer Ausführungsform das craniale Ende des Hohlstabs über die Enden der zusammengefalteten Arme der Vorrichtung hinausragen. Der Abstand, den die Arme der Vorrichtung und das craniale Ende des Hohlstabs einnehmen, ist durch die Funktion der Kombination gegeben. Das überragende Ende des cranialen Ende des Hohlstabs muß so groß ausgebildet sein, um ein ausreichendes Zurückfedern des Hohlstabs zu erlauben. Dieser Abstand darf jedoch nicht zu groß sein, da andernfalls eine zweckentsprechende Entfaltung der Arme der Vorrichtung nicht mehr möglich ist. In einem solchen Fall, indem die Vorrichtung zu tief, d.h. zu weit caudal liegend, sich entfalten muß, ist es eventuell nicht mehr möglich, daß die sich entfaltenden Arme in die Tuben des Uterus legen.

### Begriffsdefinitionen

**Cranial/Caudal:** Cranial bedeutet, daß dieser Teil zum Kopf einer Benutzerin weist, caudal besagt, daß dieser Teil vom Kopf wegweist. Die Orientierung des Gerätes ist derart, wie es bei dem Einführungsvorgang ausgerichtet ist. Somit weist die Kombination mit dem Pessar zum Kopf, wo hingegen der Stempel mit der Stempelhalterung vom Kopf wegweist. Das Einführungsgerät besteht bevorzugt aus einen für den Körper inerten Kunststoff. Das Material kann aus einer Vielzahl von Polymeren ausgewählt werden, so kann es aus Polyethylen oder Polypropylen bestehen. Das Herstellverfahren der Polymere und die Beimischungen bestimmen die Flexibilität und Weichheit des Einführungsgerätes.

**Federung:** Hierbei handelt es sich um einen Teil des Hohlstabes oder auch um einen Teil des Stempels. Als Federung können bezüglich ihrer Bauart mechanische Federn oder Federpakete geeignet sein, wie zum Beispiel ein- oder mehrgängige Schraubenfedern, wobei die Gänge auch gegenläufig mit oder ohne Knotenstellen sein können. Ferner sind Tellerfederpakete denkbar, die gegebenenfalls aus einem Stück, wie zum Beispiel einem Faltenbalg, gestaltet sind. Auch können zwischen zwei Stützringen angeordnete, eventuell leicht vorgebogene Biegestäbe verwendet werden, deren Mittellinie im wesentlichen im entspannten Zustand parallel zur Mittellinie des Hohlstabes verlaufen. Bei Belastung beulen die Biegestäbe in Richtung von der Mittellinie des Hohlstabes weg. Ebenso ist es möglich, eine Zugfederung zu verwenden die bei Bewegung des Stempels gegenüber dem Hohlstab gespreizt wird. Prinzipiell ist nicht von Bedeutung, daß die Federung Teil des Hohlstabes oder des Stempels ist. Vielmehr ist wesentlich, daß die Relativbewegung von Hohlstab und Stempel mit Hilfe der Federung elastisch, gegebenenfalls dauernd verformbar (formverändernde Kräfteabsorbierung) nicht direkt ungefedert übertragen wird. Vorteilhaft ist es, wenn die Federung Hohlstab und Stempel miteinander verbindet. Grundsätzlich sind jedoch auch Verbinder des Hohlstabes denkbar, die zum Beispiel rohrförmig den Stempel umfassen und bei Stauchfederung caudal von der Federung angeordnet sind. Sie Übertragen die kraft von der Federung auf den Stempel. Ebenfalls ist es möglich, daß der Stempel bei einer Stauchfederung caudal einen den Stempel bewegbar und röhrenförmig umgebenden Verbinder besitzt, der die Kraft auf den Hohlstab überträgt. Durch kinematische Umkehr sind bei Zugfedern andere Verbinder notwendig, die Zugkräfte weiterleiten.

**Federzone:** Hierbei handelt es sich um eine Zone, die eine Relativbewegung von Stempel und Hohlstab ermöglicht, ohne daß der Stempel über die Vorrichtung einen Druck auf den Fundus uteri oder einen anderen Gegenstand ausübt.

**Fest miteinander verbunden:** Fest miteinander verbunden bedeutet, daß hier eine sinnvollerweise nach dem Zusammenstecken von Hohlstab und Stempel herstellbare dauerhafte Verbindung vorliegt. Dabei können Teiles des Hohlstabes und des Stempels ineinandersteckbar sein. Auch komplementäre Aus- und Einbuchtungen auf Hohlstab und Stempel können als Verbindung dienen. Fest bedeutet einen Zustand, der nach dem Zusammenstecken vorliegt.

**Gleitend:** Hohlstab und Stempel können gleitend im Haftbereich miteinander verbunden sein. Das Gleiten ist für den Zusammensteck-Vorgang vor der Benutzung sinnvoll. Bevorzugt soll das Gleiten so bezüglich der Reibung sein, daß die Reibungskraft so groß ist, daß Stempel und Hohlstab sich nicht voneinander trennen, wenn die Schwerkraft auf einen der beiden einwirkt und der andere Partner gehalten wird.

**Hohlstab:** Hierbei handelt es sich um eine Röhre, die entweder einen geschlossenen oder einen perforierten Zylindermantel besitzt. Die Perforation beinhaltet den Vorteil, daß der Hohlstab weniger aufwendig hergestellt werden kann. Spritzguß ist bei dünnen Wandungen schwer herzustellen. Wird der mittige Dorn durch seitliche Stege in der mittigen Position gehalten, so verbleiben Öffnungen nach dem Erkalten des Spritzgußteiles. Werden die Öffnung so groß gehalten, daß einerseits der Dorn problemlos fixiert werden kann und andererseits scharfe Kanten an der Manteloberfläche vermieden werden kann, so ergibt sich eine für die Herstellung und die spätere Verwendung günstige Ausgestaltung des Hohlstabes.

**Intra-uterin empfängnisverhütende Vorrichtung:** Bei der intra-uterin empfängnisverhütenden Vorrichtung handelt es sich üblicherweise um ein Pessar. Bevorzugt ist in diesem Fall ein Pessar, dessen Körper mit einer Kupferspirale umgeben ist. Jedoch sind auch andere Bauformen möglich. Das Pessar selbst besteht bevorzugt aus einem elastischen Kunststoff, die Enden der Arme und das caudale Ende des Körpers sind abgerundet, um Verletzungen zu vermeiden. Der Befestigungsteil besitzt ein Auge, durch das bevorzugt zwei Kunststoffäden gezogen sind. Diese dienen dazu, das Pessar wieder aus dem Uterus entfernen zu können. Der Befestigungsteil hat weiterhin die Funktion, den Druck, der von dem Stempel ausgeübt wird, auf den gesamten Körper und die Arme zu übertragen.

### Weitere Ausführungsformen

Vorteilhaft ist es, wenn die erfindungsgemäße Kombination einen Hohlstab aufweist, der wenigstens eine Stabhalterung besitzt. Diese Stabhalterung soll derart gebaut sein, daß sie es dem Arzt ermöglicht, den Hohlstab in caudaler Richtung ziehen zu können.

Eine weitere Ausführungsform der erfindungsgemäßen Kombination besteht darin, daß die Federung cranial von der mindestens einen Stabhalterung angeordnet ist. Wenn der Arzt beim Einführen dieser Kombination den Fundus uteri berührt, wird die Federung gestaucht. Durch kinematische Umkehr ist selbstverständlich auch ein Dehnen der Federung möglich. Diese Dehnung setzt jedoch eine etwas kompliziertere Ausführungsform voraus. Wenn die Federung caudal von den Stabhalterungen liegt, muß dieser caudale Bereich des Hohlstabs dem Stempel gleitend anlegen. Die Gleitreibung sollte dabei so stark sein, die Kraft aufzufangen, die aufgrund der Schwerkraft des Stempels entsteht. Die Gleitreibung sollte jedoch ein einfaches gegeneinanderbewegen von Stempel und Hohlrohr für den Arzt ermöglichen. Dieses ist erforderlich, um den Hohlstab in caudale Richtung zu bewegen. Hierbei gleitet der gesamte Hohlstab längs des Stempels und längs der intra-uterin empfängnisverhütenden Vorrichtung.

Bevorzugt ist eine Kombination, bei der die Federung caudal von der mindestens einen Stabhalterung angeordnet ist. Vorteilhaft ist diese Anordnung der Stabhalterung, weil bei der Bewegung des Hohlstabs in caudaler Richtung der Bewegungsablauf besser kontrolliert werden kann.

Besonders bevorzugt ist eine erfindungsgemäße Kombination, bei der der Hohlstab eine Stabhalterung und der Stempel zwei Stempelhalterungen besitzt, nämlich eine Zentral-Stempelhalterung und eine Lateral-Stempelhalterung,
wobei die Zentral-Stempelhalterung an dem caudalen Ende befestigt ist,
   und
wobei die Lateral-Stempelhalterung seitlich vom Stempel und cranial zu der Zentral-Stempelhalterung angeordnet ist.

Diese Ausführungsform beinhaltet den Vorteil, daß eine unerwünschte craniale Bewegung der Kombination vermieden wird, wenn der Arzt versucht, die intra-uterin empfängnisverhütende Vorrichtung zu entfalten. Die Vermeidung der Bewegung in cranialer Richtung ist elementar wichtig, da hierbei unerwünschte Rupturen des Fundus uteri auftreten können. Halten Daumen und entweder der Zeige- oder der Mittelfinger die beiden Stempelhalterungen, so kann mit dem anderen Finger (Mittelfinger oder Zeigefinger) lediglich eine Zugbewegung ausgeführt werden. Für den Menschen ist weiterhin eine Bedienung dann einfach, wenn die Majorität der Berührungspunkte statisch gehandhabt werden kann, wogegen lediglich ein Berührungspunkt sich in Bewegung befindet. Dieses ist dadurch begründet, daß wir bei Bewegungsabläufen bestrebt sind, stets den kleineren Teil gegenüber einem feststehenden größeren Teil zu bewegen. Somit trägt diese Anordnung dazu bei, die Handhabung sicherer zu gestalten; dieses wird einerseits durch einen psychologischen Effekt hervorgerufen, andererseits ist es auch aus mechanischen Gründen sinnvoll, den Stempel und das damit verbundene Pessar statisch zu halten, wohingegen der Hohlstab in caudaler Richtung bewegt wird.

Eine weitere Ausführung der erfindungsgemäßen Kombination besteht darin daß die Vorrichtung und der Innenraum wenigstens teilweise nicht zylindersymmetrisch sind. Hierdurch wird gewährleistet, daß die empfängnisverhütende Vorrichtung sich mit Ihren Armen in Richtung der Tuben des Uterus orientiert. Der Arzt kann an dem Hohlstab erkennen, in welcher Form die empfängnisverhütende Vorrichtung vor und bei dem Einlegevorgang positioniert ist. Ein ungewolltes Drehen der Vorrichtung gegenüber den Hohlstab ist hierdurch ausgeschlossen.

Eine bevorzugte Ausführungsform der erfindungsgemäßen Kombination besteht darin, daß der Hohlstab rund und der Stempel eckig ist. Dabei kann der Stempel eine dreieckige Form einnehmen. Die dreieckige Form gibt innerhalb des Hohlstabs Hohlräume frei, durch die die beiden Fäden gelegt sein können, die an dem Auge des Befestigungsteil angeordnet sind. Dadurch wird es möglich, die Fäden innerhalb des Hohlstabs verlegen zu können.

Während zuvor als Beispiele lediglich die bekannten Pessare ausdrücklich erwähnt worden sind, die unter anderem eine Kupferspirale tragen können, umfaßt die Erfindung weiterhin eine Kombination, bei der der Körper ein Behälter ist. Dieser Behälter ist dazu geeignet, Medikamente aufzunehmen, die eine empfängnisverhütende Eigenschaft besitzen. Der Vorteil, Medikamente in dieser Art zu verabreichen besteht darin, daß sie nicht systemisch sondern lokal verabreicht werden. Dieses hat zur Folge, daß neben der mechanischen Einwirkung der Spirale auch noch gleichzeitig eine medikamentöse Einwirkung erfolgt. Dadurch wird die Sicherheit der empfängnisverhütenden Vorrichtung beträchtlich erhöht. Gleichzeitig sind möglicherweise auftretende Nebenwirkungen erheblich reduziert.

Bevorzugt ist eine Kombination, bei der der Behälter wenigstens eine semipermeablen Wand aufweist. Diese semipermeable Wand ist in der Lage, z.B. eine empfängnisverhütende Substanz allmählich freizugeben. Hierbei ist wünschenswert, daß die Substanz nicht innerhalb einer kurzen Zeitspanne freigesetzt wird, sondern daß der Behälter eine Depotwirkung hervorruft. Eine solche Lösung kann auch durch eine erfindungsgemäße Kombination bewirkt werden, bei der der Behälter wenigstens eine Öffnung aufweist. Durch diese Öffnung kann dann die empfängnisverhütende Substanz samt den galenisch üblichen Trägern und Aufnahmestoffen allmählich freigegeben werden. Diese Ausführungsform der intra-uterin empfängnisverhütenden Vorrichtung eröffnet einen neuen Verwendungsbereich. Das Einführungsgerät der Kombination benötigt keine konstruktiv bedingten Veränderungen, wenn unterschiedliche Vorrichtungen verwendet werden.

Um Verletzungen des Uterus zu vermeiden, ist es sinnvoll, daß die Arme der Vorrichtung an ihren Enden Verdickungen besitzen.

Vorteilhaft ist es, daß (i) die Stempelhalterung und (ii) die Stabhalterung oder die Stabhalterungen teilkreisförmig sind. Hierdurch wird gewährleistet, daß eine Druckrichtung in cranialer Richtung mittels der Stabhalterung nicht mehr möglich ist.

Eine bevorzugte Ausführungsform der erfindungsgemäßen Kombination besteht darin, daß die Federung aus Streben des Hohlstabs mit Strebenmittelpunkten bestehen, wobei die Strebenmittelpunkte sich bei Druck in Längsrichtung des Hohlstabs und im Zentrum des Hohlstabs wegbewegen. Durch Aussparungen des Hohlstabs in Höhe der Federung kann somit Material des Hohlstabs als federnde Streben verwendet werden. Auch wenn die bevorzugte Form darin besteht, daß sich die Strebenmittelpunkte bei Druck vom Hohlstab entfernen, ist auch eine Variante möglich, bei der die obere und untere Hälfte der Streben sich in entgegengesetzter Torsionsbewegung bewegen können, wenn der craniale und caudale Bereich des Hohlstabs gestaucht wird.

Um das Einführungsgerät möglichst mit kleinem Durchmesser herzustellen, ist es Vorteilhaft, daß die Arme der intra-uterin empfängnisverhütenden Vorrichtung im wesentlichen mit ihren Verdickungen in Aussparungen des Hohlstabs angeordnet sind.

Weiterhin ist es vorteilhaft für eine Kombination der zuvor genannten Art, wenn die Aussparungen im Inneren des Hohlstabs angeordnet sind und nicht zylinderymmetrisch sind. Dieses führt dazu, daß die Vorrichtung sich gegenüber dem Hohlstab nicht verdrehen kann, so daß der Arzt dem Hohlstab äußerlich ansieht, welche Position die Arme bei dem Entfaltungsvorgang einnehmen werden. Somit ist eine sichere Positionierung der Arme in Richtung der Tuben des Uterus gewährleistet.

Zur Erfindung umfaßt weiterhin eine Ausführungsform, bei der die Federung von dem Stempel gebildet wird. Diese Federung besitzt lediglich einen kleinen Stauchungsbereich. Die Federung hat die Funktion eines Indikators. Nach Bewegung der Federung wird der Hohlstab in caudale Richtung gezogen, wobei die Federung in ihrer maximalen Stauchung verbleibt. Der Hohlstab besitzt entweder im Bereich der Federung des Stempels eine Aussparung oder er endet deutlich cranial von der Federung. Die Federzone liegt in diesem Fall vor den Verdickungen der Vorrichtung.

### Beschreibung der Zeichnungen

In den Zeichnungen sind einige konkrete Ausführungsformen der erfindungsgemäßen Kombination dargestellt. Es zeigen im einzelnen die Figuren folgendes:
- Fig. 1: zeigt einen Längsschnitt durch eine erfindungsgemäße Kombination die eine Lateral-Stempelhalterung besitzt.
- Fig. 2: zeigt einen Längsschnitt durch eine weitere erfindungsgemäße Kombination, die zwei Stabhalterungen aufweist.
- Fig. 3: zeigt eine erfindungsgemäße Kombination im Längsschnitt, bei der die Federung cranial zu den Stabhalterungen angeordnet sind.
- Fig. 4: zeigt einen Querschnitt durch den Hohlstab, den Stempel und zwei Fäden.
- Fig. 5: zeigt einen Querschnitt durch eine weitere erfindungsgemäße Ausführungsform, wobei ein runder Hohlstab und ein eckiger Stempel abgebildet sind.
- Fig. 6: zeigt einen Längsschnitt durch eine erfindungsgemäße Vorrichtung mit Behälter.
- Fig. 7: zeigt einen Längsschnitt durch eine erfindungsgemäße Vorrichtung, deren Behälter eine Öffnung und eine semipermeable Wand besitzt.
- Fig. 8: zeigt einen Längsschnitt durch eine Kombination, bei der die Verdickungen der Enden der Arme in Aussparungen im Innern des Hohlstabs liegen.
- Fig. 9: zeigt einen Längsschnitt durch eine erfindungsgemäße Kombination bei der die Federzone cranial zur Vorrichtung angeordnet ist.
- Fig. 10: zeigt eine bevorzugte Ausführungsform, wobei links die vorrichtung eingezogen ist und rechts fast vollständig ausgestoßen ist.

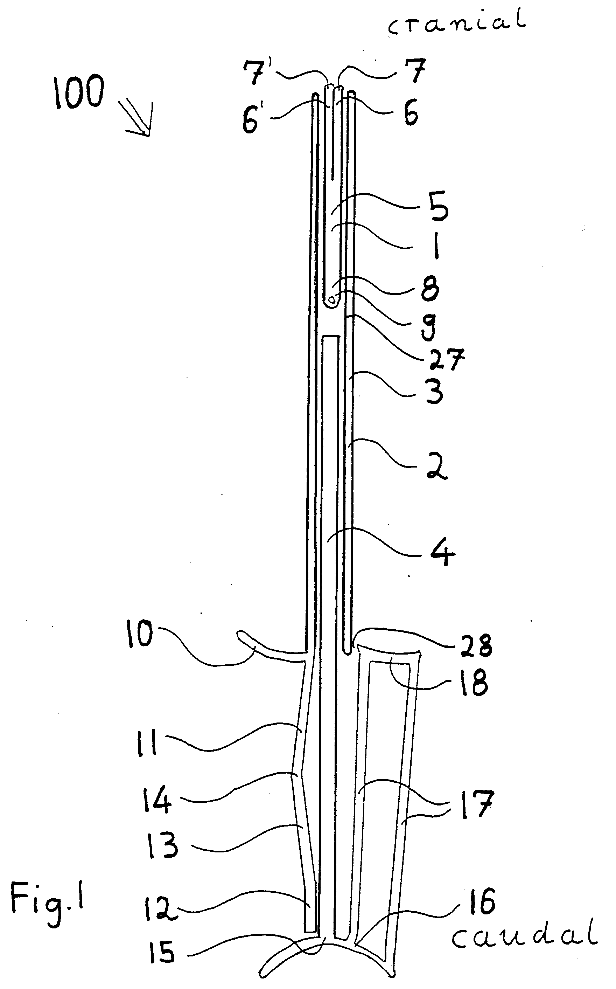

Das Verhältnis von Höhe zur Breite ist bei den Längsschnitten der Figuren 1 bis 9 nicht maßstabsgetreu. Aufgrund der klareren Zeichenweise sind die Teile der Kombination bezüglich der Breite übertrieben dargestellt.

Die Kombination in der Figur 1 besteht aus einer Vorrichtung 1, einem Einführungsgerät 2, das aus einem Hohlstab 3 und einem Stempel 4 besteht. Der Hohlstab 3 umgibt sowohl den Stempel 4 als auch teilweise die Vorrichtung 1.

Die intra-uterin empfängnisverhütende Vorrichtung 1 besteht aus einem Körper 5, an dessen cranialen Ende sich zwei Arme 6 und 6' befinden, deren Ende in Verdickungen 7 und 7' auslaufen. Der caudale Teil des Körpers 5 wird von einem Befestigungsteil 8 gebildet, der ein Auge 9 besitzt. Durch dieses Auge 9 werden zwei Kunststoffäden, die in der Figur nicht abgebildet sind, gezogen. Diese Kunststoffäden verlaufen in dem Hohlstab bis zu dem caudalen Ende des Hohlstabs.

Der Hohlstab 3 weist an seinem caudalen Ende eine Stabhalterung 10 auf, die bei sachgemäßer Bedienung von dem Zeigefinger des Arztes betätigt wird. Etwas caudal von der Stabhalterung entfernt geht der Hohlstab 3 in die Federung 11 über, die sich bis zu dem caudalen Ende des Hohlstabs erstreckt, der als ein Haftbereich 12 ausgebildet ist. Die Gleitreibung im Haftbereich ist so stark ausgeprägt, daß ein Verschieben des Stempels auf Grund der Schwerkraft nicht erfolgt. Der Hohlstab 3 besitzt in der Federung 11 Streben 13, die so ausgebildet sind, daß sie bei einem Druck des Hohlstabs in Richtung des Haftbereiches 12 dazu führen, sich in ihrem Mittelpunkt von der Hohlstabsachse zu entfernen. Dieser Mittelpunkt wird von einem Strebenmittelpunkt 14 gebildet, der eine Verarbeitung der Strebe umfaßt, die ein V-förmiges Verformen der sonst fast linearen Strebe 13 zur Folge hat. Die Ausmaße der Streben 13 (lediglich eine Strebe 13 ist in der Figur zu sehen) ist so dimensioniert, daß bei einem leichten Druck auf das craniale Ende des Hohlstabs sich der Strebenmittelpunkt 14 von der Achse des Hohlstabs 3 entfernt.

Der Haftbereich 12 liegt einer Stempelhalterung 15 auf, die sich an dem caudalen Ende des Stempels 4 befindet. Bei dieser Stempelhalterung handelt es sich um eine Zentral-Stempelhalterung 16, die bei sachgemäßer Bedienung von dem Daumen des Arztes gehalten wird. Teile dieser Zentral-Stempelhalterung die in eine Tragvorrichtung 17 über, die aus zwei Streben besteht. Diese Tragvorrichtung 17 erstreckt sich bis zu einer Lateral-Stempelhalterung 18, die bei entspannter Federung 11 auf gleicher Höhe wie die Stabhalterung 10 liegt. Bei sachdienlicher Bedienung liegt der Mittelfinger der Lateral-Stempelhalterung an. Die Tragvorrichtung 17 besitzt an seiner zum Hohlstab gelegenen Seite einen Gleitzusatzbereich 28, der eine Gleitbewegung zwischen Gleitzusatzbereich und Außenseite des Hohlstabes 3 zur Folge hat.

Zwischen dem cranialen Ende des Stempels 4 und dem caudalen Ende des Befestigungsteiles 8 liegt ein Hohlraum, der von dem Hohlstab 3 umgeben wird. Dieser Hohlraum bildet eine Federzone 27. Diese Federzone wird im Volumen verringert, wenn die zusammengefalteten Arme 6 und 6' den Fundus uteri berührt.

Die Bedienung dieses Gerätes besteht darin, daß der Arzt vor Einführung der Kombination 100 die Vorrichtung 1 samt der daran hängenden Fäden in den Hohlstab 3 einführt. Indem er die Fäden die unten aus dem Hohlstab herausschauen, festhält, schiebt er den Stempel 4 in das caudale Ende des Hohlstabs 3 ein. Diese Bewegung setzt er solange fort, bis die Federung 11 gerade anspricht. Nun ist das Gerät Einsatzbereit. Der Arzt nimmt die erfindungsgemäße Kombination und führt sie durch die Vagina und den Muttermund in den Uterus ein. Bei vorsichtiger Bedienung ist ein vorheriges Sondieren nicht unbedingt erforderlich, ein Sondieren ist jedoch aus Sicherheitsgründen wünschenswert. Der Arzt faßt die Kombination im Bereich der Tragevorrichtung 17 und der Zentral-Stempelhalterung 16 an. Ebenfalls kann er mit den Fingern auch den Haftbereich 12 berühren.

Kommt der Arzt mit dem cranialen Ende des Hohlstabs 3 an den Fundus Uterus, so wird ein Druck auf die Federung 11 ausgelöst, der dazu führt, daß der Strebenmittelpunkt 14 sich von der Achse des Hohlstabs 3 entfernt. Der Arzt weiß nun, daß er die Position für die Vorrichtung 1 erreicht hat, in der eine Entfaltung der Vorrichtung wünschenswert ist. Dieser Vorgang wird dadurch in die Wege geleitet, daß der Arzt die Stabhalterung 10 in caudale Richtung zieht, wobei die Zentral-Stempelhalterung 16 und die Lateral-Stempelhalterung 18 gegenüber der Patientin nicht bewegt werden. Wir haben es hiermit nur um eine Zugbewegung zu tun, eine Verletzungsgefahr der Patientin ist somit schier ausgeschlossen. Bei dem Herabziehen des Hohlstabs 3 werden die Arme 6 und 6', die unter Spannung in zusammengelegter Form vorliegen, entfaltet. Sie weisen dabei in Richtung der Tuben des Uterus. Wenn die Federung 11 maximal komprimiert ist, weiß der Arzt, das die Vorrichtung 1 sich außerhalb des Hohlstabs 3 befindet. Der Einlegevorgang ist somit abgeschlossen, Hohlstab und Stempel werden aus dem Uterus entfernt.

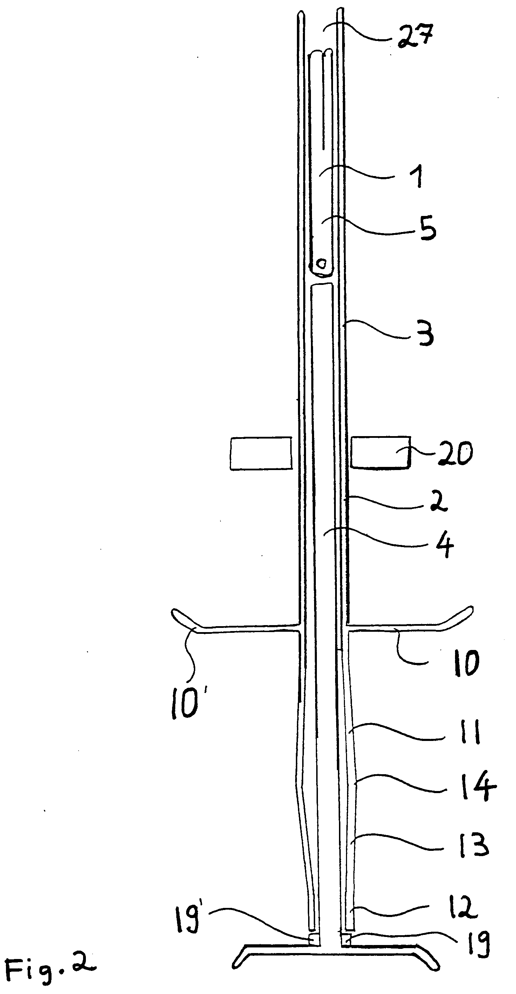

Die Figur 2 zeigt eine ähnliche Vorrichtung jedoch besitzt das Einführungsgerät 2 an dem Hohlstab 3 eine weitere Stabhalterung 10'. Somit liegen die beiden Stabhalterungen 10 und 10' paarig vor. Der Haftbereich 12 umgibt vollständig den Stempel 4. Der Haftbereich liegt dabei Auflagen 19 und 19' auf. Diese Auflagen dienen als Gegenlager für die Kräfte, die entweder auf dem cranialen Ende des Hohlstabs im Falle einer Berührung des Fundus uteri ausgeübt wird oder im Falle der Bedienung durch den Arzt durch ein Herabziehen der Stabhalterungen 10 und 10' in caudaler Richtung herbeigeführt wird. Die Figur 2 zeigt weiterhin einen hohlzylinderförmigen Schieber 20 der verschiebbar der Außenwand des Hohlstabs 3 anliegt. Dieser Schieber dient dazu, daß der Arzt die Distanz zwischen Fundus uteri und Muttermund, die er zuvor durch eine Sonde ermittelt hat, einstellen kann. Hierdurch erhält er eine weitere Sicherheit, um kontrollieren zu können, daß er das craniale Ende des Hohlstabs 3 in einem geringen Abstand vom Fundus uteri zum Stillstand bringt. Weiterhin zeigt diese Figur eine Federzone 27, die cranial von der Vorrichtung 1 liegt. Der Hohlstab 3 ragt über die Verdickungen 7 und 7' hinaus und begrenzt einen Hohlraum.

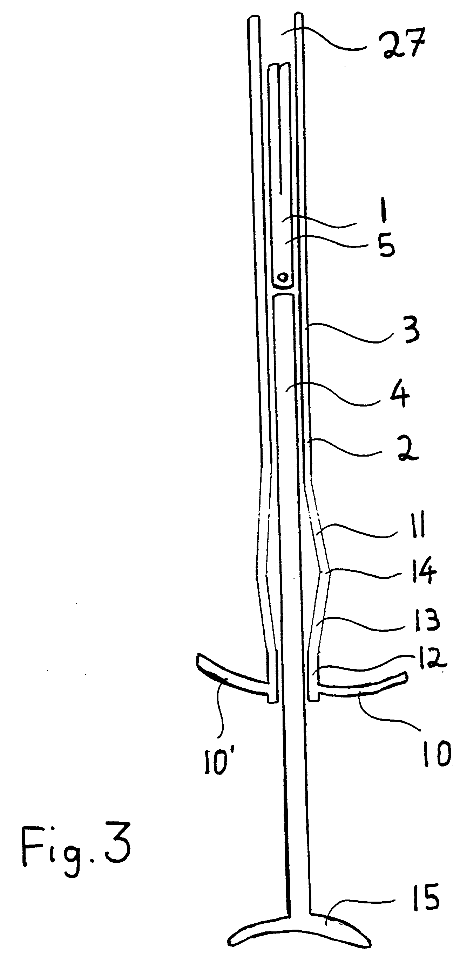

Die Figur 3 zeigt eine weitere erfindungsgemäße Vorrichtung, bei der die Federung 11 cranial von den beiden Stabhalterungen 10 und 10' liegt. Wichtig ist bei dieser Ausführungsform der Haftbereich 12, der zwischen Stempel 4 und Haftbereich 12 eine Gleitreibung aufweist, die so groß ist, daß die Federung 11 gestaucht wird und dadurch die Strebenmittelpunkte 14 sich von der Achse des Hohlstabs 3 entfernen, wenn das Einführungsgerät den Fundus uteri berührt.

Die Gleitreibung darf jedoch nicht so stark sein, daß beim Entfaltungsvorgang der Vorrichtung 1 dieser Haftbereich nicht in caudale Richtung längs des Stempels 4 gezogen werden kann.

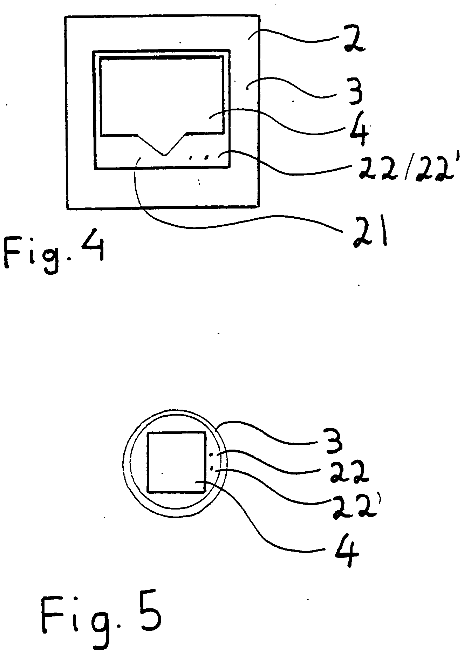

Die Figur 4 zeigt eine weitere Ausführungsform der Kombination, wobei ein Einführungsgerät 2 und Teile der Vorrichtung 1 zu sehen sind. Das Einführungsgerät 2 besteht hier aus einem quadratischen Hohlstab, in dessem Inneren sich ein rechteckiger Stempel mit dreieckiger Ausstülpung an einer der rechteckigen Wände befindet. Dadurch bildet sich ein Hohlraum 21, indem zwei Kunststoffäden 22 und 22' verlaufen. Dieser Hohlraum ist für gerade diese Kunststoffäden gedacht, damit diese sich bei dem Entfernen des Einführungsgerätes aus dem Uterus nicht festhaften.

Die Figur 5 zeigt eine weitere Ausführungsform, wobei der Hohlstab 3 kreisförmig ist und der Stempel 4 viereckig ausgebildet ist. Auch in dieser Figur sind die beiden Kunststoffäden 22 und 22' zu sehen.

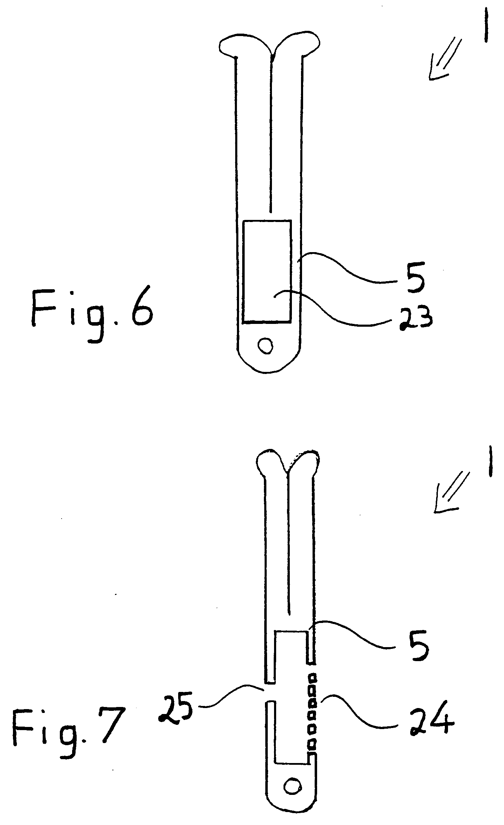

Die Figur 6 zeigt eine Vorrichtung 1 mit einem Körper 5, in dessen Mittelpunkt sich ein Behälter 23 befindet. Dieser Behälter ist z.B. zur Aufnahme von Medikamenten geeignet.

Figur 7 zeigt eine Vorrichtung 1 mit einem Behälter, der auf der rechten Seite der Vorrichtung eine semipermeable Wand 24 und auf der linken Seite der Vorrichtung eine Öffnung des Behälters besitzt. So kann entweder durch die semipermeable Wand oder durch die Öffnung 25 ein in dem Behälter sich befindliche Substanz in das umliegende Medium diffundieren. Eine allmähliche Freigabe des Medikaments ist dadurch ermöglicht.

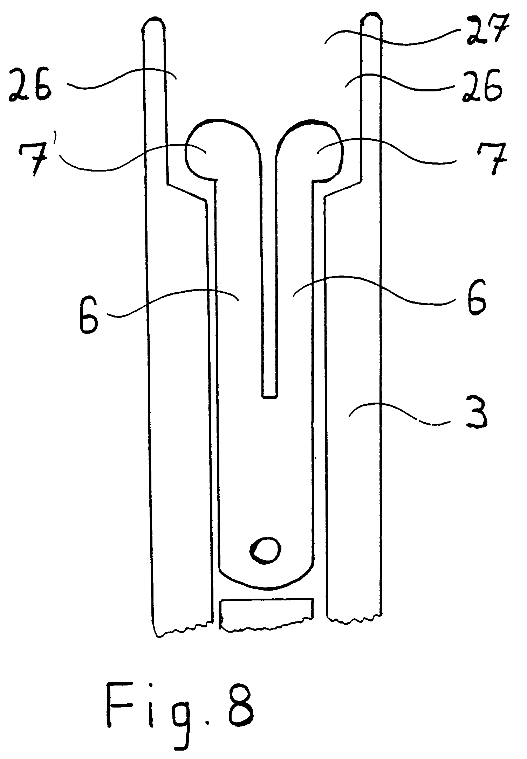

Die Figur 8 zeigt eine Kombination gemäß der Erfindung, bei der die Verdickungen 7 und 7' an den Armen 6 und 6' in Aussparungen 26 und 26' des Hohlstabs 3 liegen. Diese Aussparungen, die nicht drehsymmetrisch ausgebildet sind, gewährleisten, daß die Vorrichtung bezüglich des Hohlstabs in einer bestimmten Drehung vororientiert ist. Somit sieht der behandelnde Arzt an dem Hohlstab 3 äußerlich, in welcher Form die Arme 6 und 6' der Vorrichtung 1 sich befinden. Hierdurch wird gewährleistet, daß bei dem Entfalten der Arme 6 und 6' eine richtige Positionierung bezüglich der Tuben des Uterus gewährleistet ist.

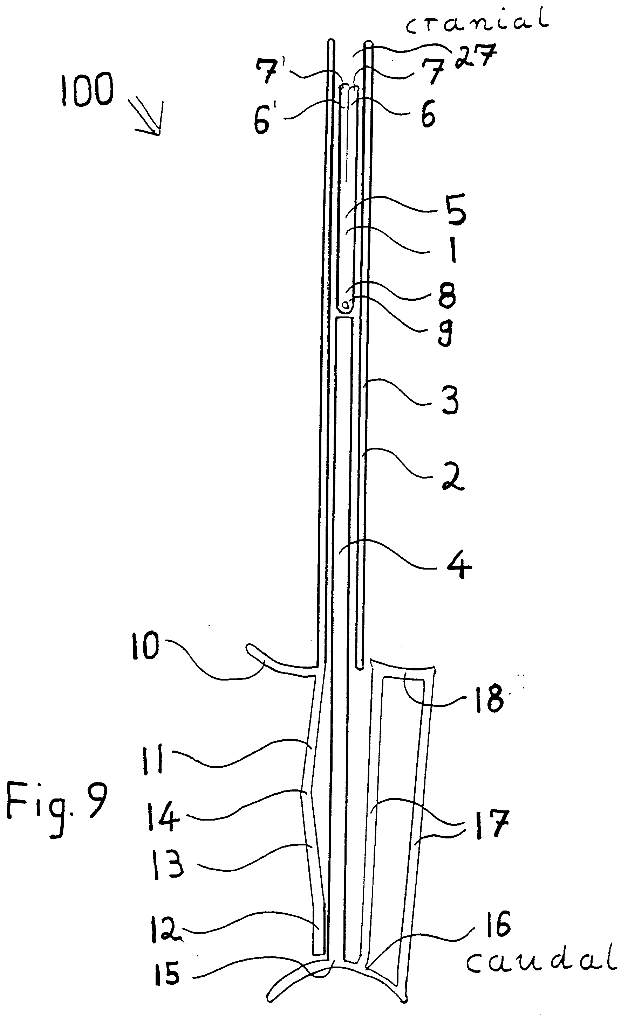

Figur 9 zeigt eine weitere Ausführungsform der erfindungsgemäßen Kombination, bei der die Federzone 27 cranial zur Vorrichtung angeordnet ist.

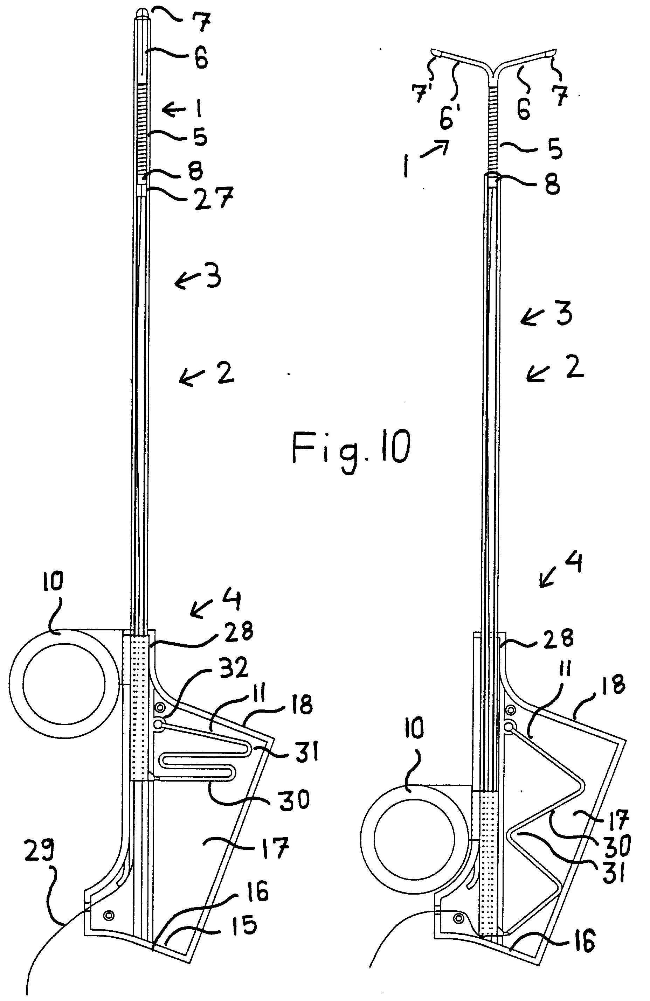

Die Figuren 10 und 11 stellen die bevorzugte Ausführungsform dar.
Die Kombination in der Figur 10 besteht aus einer Vorrichtung 1, einem Einführungsgerät 2, das aus einem Hohlstab 3 und einem Stempel 4 besteht. Der Hohlstab 3 umgibt sowohl den Stempel 4 als auch teilweise die Vorrichtung 1.

Die intra-uterin empfängnisverhütende Vorrichtung 1 besteht aus einem Körper 5, an dessen cranialen Ende sich zwei Arme 6 und 6' befinden, deren Ende in Verdickungen 7 und 7' auslaufen. Der caudale Teil des Körpers 5 wird von einem Befestigungsteil 8 gebildet, der ein Auge besitzt. Durch dieses Auge werden zwei Kunststoffäden 29 gezogen. Diese Kunststoffäden verlaufen in dem Hohlstab und dem Stempel bis zu deren caudalen Enden.

Der Hohlstab 3 weist an seinem caudalen Ende eine Stabhalterung 10 auf, die bei sachgemäßer Bedienung von dem Zeigefinger des Arztes betätigt wird. Bei in den Hohlstab eingezogener Vorrichtung liegt die Federung 11 in Form einer Zugfeder vor, die sich von dem caudalen Ende des Hohlstabs erstreckt. Der Hohlstab 3 besitzt in der Federung 11 mäanderförmig angeordnete Stege 30, die über Biegezonen 31 verbunden sind. Die Ausmaße der Stege und der Biegezonen ist so dimensioniert, daß bei einem leichten Druck auf das craniale Ende die Federung gestreckt wird. Die dabei auftretende Energie wird in plastische Energie umgewandelt. Die Federung 11 ist in ihrer vom Ansatzpunkt am Hohlstab 3 wegweisenden Ende an einer Öse 32 befestigt, die Teil des Stempels 4 ist.

Der Hohlstab 3 hat freies Spiel gegenüber dem Stempel 4. Im caudalen Bereich umragt der Stempel 4 den caudalen Bereich des Hohlstabes 3. Dabei bildet der Stempel 4 eine Stempelhalterung 15, die sich an dem caudalen Ende des Stempels 4 befindet. Bei dieser Stempelhalterung handelt es sich um eine Zentral-Stempelhalterung 16, die bei sachgemäßer Bedienung von dem Daumen des Arztes gehalten wird. Diese Zentral-Stempelhalterung 16 geht in eine Tragvorrichtung 17 über, die einen Hohlkörper bildet, der vor allem die Federung 11 und den Hohlstab 4 umgibt. besteht. Diese Tragvorrichtung 17 erstreckt sich bis zu einer Lateral-Stempelhalterung 18, die bei entspannter Federung 11 etwa auf gleicher Höhe wie die Stabhalterung 10 liegt. Bei sachdienlicher Bedienung liegt der Mittelfinger der Lateral-Stempelhalterung an. Die Tragvorrichtung 17 besitzt an seiner zum Hohlstab gelegenen Seite einen Gleitzusatzbereich 28, der eine Gleitbewegung zwischen Gleitzusatzbereich und Außenseite des Hohlstabes 3 zur Folge hat.

Zwischen dem cranialen Ende des Stempels 4 und dem caudalen Ende des Befestigungsteiles 8 liegt ein Hohlraum, der von dem Hohlstab 3 umgeben wird. Dieser Hohlraum bildet eine Federzone 27. Diese Federzone wird im Volumen verringert, wenn die zusammengefalteten Arme 6 und 6' den Fundus uteri berührt und der Stempel 4 im Hohlstab 3 nach cranial bewegt wird.

### Bezugszeichenliste:

- 100: Kombination
- 1: Vorrichtung
- 2: Einführungsgerät
- 3: Hohlstab
- 4: Stempel
- 5: Körper
- 6, 6': Arm
- 7, 7': Verdickungen der Arme
- 8: Befestigungsteil
- 9: Auge
- 10, 10': Stabhalterung
- 11: Federung
- 12: Haftbereich
- 13: Streben
- 14: Strebenmittelpunkt
- 15: Stempelhalterung
- 16: Zentral-Stempelhalterung
- 17: Tragvorrichtung
- 18: Lateral-Stempelhalterung
- 19, 19': Auflage
- 20: Schieber
- 21: Hohlraum
- 22, 22': Kunststoffäden
- 23: Behälter
- 24: semipermeable Wand
- 25: Öffnung des Behälters
- 26, 26': Aussparungen des Hohlstabs
- 27: Federzone
- 28: Gleitzusatzbereich
- 29: Kunststoff-Fäden
- 30: Stege
- 31: Biegezone
- 32: Öse

## Patentansprüche

1. Kombination (100) aus einer intra-uterin empfängnisverhütenden Vorrichtung (1) und einem dieser Vorrichtung (1) entsprechenden Einführungsgerät (2) mit folgenden Merkmalen:
a) die intra-uterin empfängnisverhütende Vorrichtung (1) besitzt einen Körper (5), an dessen cranialem Ende wenigstens zwei elastische Arme (6, 6') angeordnet sind und dessen caudales Ende ein Befestigungsteil (8) bildet, an das eine Vorrichtung zur Entfernung der intra-uterin empfängnisverhütenden Vorrichtung (1) aus dem Uterus befestigbar ist;
b) das Einführungsgerät (2) besteht aus einem Hohlstab (3) und einem im Hohlstab (3) bewegbaren Stempel (4), der am caudalen Ende wenigstens eine Stempelhalterung (15) aufweist;
c) im caudalen Bereich ist der Hohlstab (3) und/oder der Stempel (4) als Federung ausgebildet;
d) am caudalen Ende des Hohlstabs (3) und im caudalen Bereich des Stempels (4) cranial von der Stempelhalterung (15) sind Hohlstab (3) und Stempel (4) in einem Haftbereich (12) gleitend oder fest miteinander verbunden;
e) zwischen Stempel (4) und intra-uterin empfängnisverhütender Vorrichtung (1) und/oder cranial von der vollständig in den Hohlstab (3) eingezogenen intra-uterin empfängnisverhütenden Vorrichtung (1) befindet sich ein in radialer Richtung von dem Hohlstab (3) umgebener Hohlraum als Federzone (27) innerhalb der bei einer Relativbewegung von Stempel (4) und Hohlstab (3) zueinander die Federung des Hohlstabs (3) anspricht und die intra-uterin empfängnisverhütende Vorrichtung (1) noch vollständig im Einführungsgerät (2) bleibt.

2. Kombination nach Anspruch 1, bei der der Hohlstab (3) wenigstens eine Stabhalterung (10) besitzt.

3. Kombination nach Anspruch 1 oder 2, bei der die Federung (11) cranial von der mindestens einen Stabhalterung (10, 10') angeordnet ist.

4. Kombination nach Anspruch 1 oder 2, bei der die Federung (11) caudal von der mindestens einen Stabhalterung (10, 10') angeordnet ist.

5. Kombination nach einem der vorherigen Ansprüche, bei der der Hohlstab (3) eine Stabhalterung (10) und der Stempel (4) zwei Stempelhalterungen (15) besitzt, nämlich eine Zentral-Stempelhalterung (16) und eine Lateral-Stempelhalterung (18),
wobei die Zentral-Stempelhalterung an dem caudalen Ende befestigt ist,
und
wobei die Lateral-Stempelhalterung seitlich vom Stempel (4) und cranial zu der Zentral-Stempelhalterung angeordnet ist.

6. Kombination nach einem der vorherigen Ansprüche, bei der (i) die Stempelhalterung (15) und (ii) die Stabhalterung oder die Stabhalterungen (10, 10') teilkreisförmig sind.

7. Kombination nach einem der vorherigen Ansprüche, bei der die Federung (11) aus Streben (13) des Hohlstabs (3) mit Strebenmittelpunkten (14) bestehen, wobei die Strebenmittelpunkte sich bei Druck in Längsrichtung des Hohlstabs von dem Zentrum des Hohlstabs wegbewegen.

8. Kombination nach einem der vorherigen Ansprüche, bei der die Vorrichtung (1) und der Innenraum des Hohlstabs (3) wenigstens teilweise nicht zylindersymmetrisch sind.

9. Kombination nach einem der vorherigen Ansprüche 1 bis 7, bei der der Hohlstab (3) rund und der Stempel (4) eckig ist.

10. Kombination nach einem der vorherigen Ansprüche, bei der der Körper (5) ein Behälter (23) ist.

11. Kombination nach Anspruch 10, bei der der Behälter (23) wenigstens eine semipermeablen Wand (24) aufweist.

12. Kombination nach einem der vorherigen Ansprüche 10 und 11 dadurch gekennzeichnet, daß der Behälter (23) wenigstens eine Öffnung (25) aufweist.

13. Kombination nach einem der vorherigen Ansprüche, bei der die Arme (6 und 6') der Vorrichtung (1) an ihren Enden Verdickungen (7 und 7') besitzen.

14. Kombination nach einem der vorherigen Ansprüche 11 bis 13, bei der die Arme (6 und 6') der intra-uterin empfängnisverhütenden Vorrichtung (1) im wesentlichen mit ihren Verdickungen (7 und 7') in Aussparungen (26) des Hohlstabs (3) angeordnet sind.

15. Kombination nach Anspruch 14, bei der die Aussparungen (26 und 26') im Innern des Hohlstabs (3) angeordnet sind und nicht zylindersymmetrisch sind.

## Claims

1. Combination (100) of an intra-uterine contraceptive device (1) and an insertion instrument (2) appropriate for that device (1) having the following features :
a) the intrauterine contraceptive device (1) comprises a body (5) at the cranial end of which there are arranged at least two resilient arms (6, 6') and at the caudal end of which a fastening means (8) is formed to which a means for removing the intra-uterine contraceptive device (1) from the uterus can be fixed;
b) the insertion instrument (2) consists of a hollow rod (3) and a plunger (4) movable in the hollow rod (3), which plunger has at least one plunger-holding means (15) at the caudal end;
c) in the caudal region the hollow rod (3) and/or the plunger (4) comprise a spring arrangement;
d) at the caudal end of the hollow rod (3) and in the caudal region of the plunger (4), cranially from the plunger-holding means (15), the hollow rod (3) and the plunger (4) are joined to one another fixedly or in a sliding manner in an engaging region (12);
e) between the plunger (4) and the intra-uterine contraceptive device (1) and/or cranially from the intra-uterine contraceptive device (1) when fully inserted in the hollow rod (3) there is a hollow space, surrounded in the radial direction by the hollow rod (3), as a buffer zone (27) within which, on relative movement of the plunger (4) and hollow rod (3) with respect to one another, the spring arrangement of the hollow rod (3) responds and the intra-uterine contraceptive device (1) still remains fully in the insertion instrument (2).

2. Combination according to claim 1, in which the hollow rod (3) has at least one rod-holding means (10).

3. Combination according to claim 1 or 2, in which the spring arrangement (11) is arranged cranially from the at least one rod-holding means (10, 10').

4. Combination according to claim 1 or 2, in which the spring arrangement (11) is arranged caudally from the at least one rod-holding means (10, 10').

5. Combination according to any one of the preceding claims, in which the hollow rod (3) has a rod-holding means (10) and the plunger (4) has two plunger-holding means (15), that is, a central plunger-holding means (16) and a lateral plunger-holding means (18),
wherein the central plunger-holding means is fastened to the caudal end and
wherein the lateral plunger-holding means is arranged laterally from the plunger (4) and cranially with respect to the central plunger-holding means.

6. Combination according to any one of the preceding claims, in which (i) the plunger-holding means (15) and (ii) the rod-holding means (10, 10') are of partially circular shape.

7. Combination according to any one of the preceding claims, in which the spring arrangement (11) consists of braces (13) of the hollow rod (3) having brace mid-points (14), the brace mid-points being moved away from the centre of the hollow rod when pressure is exerted in the longitudinal direction of the hollow rod.

8. Combination according to any one of the preceding claims, in which the device (1) and the interior of the hollow rod (3) are at least in part not cylindrically symmetrical.

9. Combination according to any one of the preceding claims 1 to 7, in which the hollow rod (3) is round and the plunger (4) is angular.

10. Combination according to any one of the preceding claims, in which the body (5) provides a container (23).

11. Combination according to claim 10, in which the container (23) has at least one semi-permeable wall (24).

12. Combination according to either claim 10 or claim 11, characterised in that the container (23) has at least one aperture (25).

13. Combination according to any one of the preceding claims, in which the arms (6 and 6') of the device (1) have thickened regions (7 and 7') at their ends.

14. Combination according to any one of the preceding claims 11 to 13, in which the arms (6 and 6') of the intra-uterine contraceptive device (1) are arranged essentially with their thickened regions (7 and 7') in recesses (26) in the hollow rod (3).

15. Combination according to claim 14, in which the recesses (26 and 26') are arranged on the inside of the hollow rod (3) and are not cylindrically symmetrical.

## Revendications

1. Ensemble combiné (100) formé d'un dispositif contraceptif intra-utérin (1) et d'un appareil d'introduction (2) correspondant à ce dispositif (1), présentant les caractéristiques suivantes :
a) le dispositif contraceptif intra-utérin (1) possède un corps (5), sur l'extrémité sommitale duquel sont disposés au moins deux bras élastiques (6,6') et dont l'extrémité caudale forme une partie de fixation (8), à laquelle peut être fixé un dispositif servant à retirer de l'utérus le dispositif contraceptif intra-utérin (1);
b) l'appareil d'introduction (2) est constitué par une tige creuse (3) et par un piston (4) déplaçable dans la tige creuse (3) et qui comporte au moins un dispositif de retenue (15) au niveau de l'extrémité caudale;
c) dans la partie caudale, la tige creuse (3) et/ou le piston (4) est/sont agencé(s) sous la forme d'une suspension;
d) au niveau de l'extrémité caudale de la tige creuse (3) et dans la partie caudale du piston (4), vers le côté sommital par rapport au dispositif (15) de retenue du piston, la tige creuse (3) et le piston (4) sont reliés entre eux de manière à glisser ou de façon fixe dans une zone d'adhérence (12);
e) entre le piston (4) et le dispositif contraceptif intra-utérin (1) et/ou sur le côté sommital par rapport au dispositif contraceptif intra-utérin (1) complètement inséré dans la tige creuse (3) est présente une cavité entourée dans la direction radiale par la tige creuse (3) et constituant une zone formant ressort (27), à l'intérieur de laquelle, dans le cas d'un déplacement relatif réciproque du piston (4) et de la tige creuse (3), la suspension de la tige creuse (3) répond et le dispositif contraceptif intra-utérin (1) reste encore complètement dans l'appareil d'introduction (2).

2. Ensemble combiné selon la revendication 1, dans lequel la tige creuse (3) possède au moins un dispositif (10) de retenue de la tige.

3. Ensemble combiné selon la revendication 1 ou 2, dans lequel la suspension (11) est disposée sur le côté sommital par rapport à au moins un dispositif (10,10') de retenue de la tige.

4. Ensemble combiné selon la revendication 1 ou 2, dans lequel la suspension (11) est disposée sur le côté caudal par rapport à au moins un dispositif (10,10') de retenue de la tige.

5. Ensemble combiné selon l'une quelconque des revendications précédentes, dans lequel la tige creuse (3) comporte un dispositif (10) de retenue de la tige, et le piston (4) comporte deux dispositifs (15) de retenue du piston, a savoir un dispositif central (16) de retenue du piston et un dispositif latéral (18) de retenue du piston,
le dispositif central de retenue du piston étant situé sur l'extrémité caudale, et
le dispositif latéral de retenue du piston étant disposé latéralement par rapport au piston (4) et sur le côté sommital par rapport au dispositif central de retenue du piston.

6. Ensemble combiné selon l'une des revendications précédentes, dans lequel (i) le dispositif (15) de retenue du piston et (ii) le dispositif de retenue de la tige ou les dispositifs (10,10') de retenue de la tige ont une forme partiellement circulaire.

7. Ensemble combiné selon l'une des revendications précédentes, dans lequel la suspension (11) est formée d'entretoises (13) de la tige creuse (3), comportant des points médians (14), les points médians des entretoises s'écartant du centre de la tige creuse dans le cas d'une pression exercée dans la direction longitudinale de la tige creuse.

8. Ensemble combiné selon l'une des revendications précédentes, dans lequel le dispositif (1) et l'espace intérieur de la tige creuse (3) ne possèdent pas, au moins partiellement, une symétrie cylindrique.

9. Ensemble combiné selon l'une des revendications précédentes 1 à 7, dans lequel la tige creuse (3) est ronde et le piston (4) est anguleux.

10. Ensemble combiné selon l'une des revendications précédentes, dans lequel le corps (5) est un récipient (23).

11. Ensemble combiné selon la revendication 10, dans lequel le récipient (23) comporte au moins une paroi semi-perméable (24).

12. Ensemble combiné selon l'une des revendications précédentes 10 et 11, caractérisé en ce que le récipient (23) comporte au moins une ouverture (25).

13. Ensemble combiné selon l'une des revendications précédentes, dans lequel les bras (6 et 6') du dispositif (1) possèdent les épaississements (7 et 7') au niveau de leurs extrémités.

14. Ensemble combiné selon l'une des revendications précédentes 11 à 13, dans lequel les bras (6 et 6') du dispositif contraceptif intra-utérin (1) sont disposés essentiellement, par leurs épaississements (7 ou 7'), dans des évidements (26) de la tige creuse (3).

15. Ensemble combiné selon la revendication 14, dans lequel les évidements (26 et 26') sont aménagés à l'intérieur de la tige creuse (3) et ne présentent aucune symétrie cylindrique.
